(19) Europäisches Patentamt

European Patent Office

Office européen des brevets

(11) Veröffentlichungsnummer: **0 469 245 A1**

# (12) EUROPÄISCHE PATENTANMELDUNG

(21) Anmeldenummer: **91106999.5**

(51) Int. Cl.⁵: **A61B 17/12**

(22) Anmeldetag: **30.04.91**

(30) Priorität: **30.07.90 DE 4024106**

(43) Veröffentlichungstag der Anmeldung:
**05.02.92 Patentblatt 92/06**

(84) Benannte Vertragsstaaten:
**AT BE CH DE DK ES FR GB GR IT LI NL SE**

(71) Anmelder: **ETHICON GmbH & Co. KG**
**Robert-Koch-Strasse 1**
**W-2000 Norderstedt(DE)**

(72) Erfinder: **Troidl, Hans, Prof. Dr.-Med.**
**Ostmerheimer Strasse 200**
**W-5000 Köln 91(DE)**

Erfinder: **Heidmüller, Harald**
**Heidenrichstrasse 10**
**W-5000 Köln 80(DE)**
Erfinder: **Al-Jaziri, Ahmad, Dr.**
**In der Rozenau 8**
**W-5000 Köln 90(DE)**
Erfinder: **Kaufmann, Helmut**
**Giessstrasse 9**
**W-7200 Tuttlingen(DE)**

(74) Vertreter: **UEXKÜLL & STOLBERG**
**Patentanwälte**
**Beselerstrasse 4**
**W-2000 Hamburg 52(DE)**

(54) **Klammersetzgerät für Ligatur-Clips.**

(57) Die Erfindung betrifft ein Klammersetzgerät für Ligatur-Clips, insbesondere für laparoskopische Operationen, bestehend aus einem rohrartigen Gestänge, an dessen einem Ende ein zweiteiliger scherenartiger Griff und an dessem entgegengesetzten Ende Klemmbacken zur Aufnahme einer Klammer vorgesehen sind, wobei der eine Griffteil des scherenartigen Griffs fest mit dem einen Teil des Gestänges verbunden ist, während der andere Griffteil ein Schließen der Klemmbacken bewirkt, wenn beide Griffteile um ihre unterhalb und senkrecht zur Längsachse des rohrartigen Gestänges liegende Schwenkachse gegen Federdruck relativ zueinander bewegt werden, welcher dadurch gekennzeichnet ist, daß in dem rohrartigen Gestänge (2) ein Rückholdraht (30) bewegbar angeordnet ist, dessen vorderes bzw. proximales Ende (32) hakenförmig ausgebildet ist und in Ruhestellung mit nach oben offenem Haken neben den Klemmbacken (24, 26) liegt, während das hintere bzw. distale Ende (34) des Rückholdrahtes (30) im Bereich des Griffes (4) mit einem aus dem rohrartigen Gestänge (2) herausragenden Betätigungshebel (36) verbunden ist.

FIG. 1

Die Erfindung betrifft ein Klammersetzgerät für Ligatur-Clips bzw. einen endoskopischen Clip-Applikator, insbesondere für laparoskopische chirurgische Operationen gemäß Oberbegriff des Hauptanspruches.

Derartige Klammersetzgeräte werden z.B. gemäß Akt. Urol. 15 (1984), S. 126 bis 128, z.B. in einer Ausführung gemäß Firmenblatt OP-INST 9 A der Firma Storz Ausgabe 5(89) eingesetzt, um Gefäße wie z.B. eine Aorta mit Clips oder Klammern aus Metall wie Silber oder Titan aber auch aus resobierbarem Material zu ligieren.

Wenngleich der Einsatz von Clips wegen ihrer schnellen Anwendung und Zugänglichkeit bei schwer zugänglichen Gefäßen statt der herkömmlichen Ligaturtechnik mit Nahtmaterial in vielen Bereichen der Chirurgie gut eingeführt ist, ergeben sich bei laparoskopischer Chirurgie mit den bislang bekannten Klammersetzgeräten Schwierigkeiten insofern, als das Einfangen der zu ligierenden Gefäße und das Einführen dieser in den Klammerbereich nicht von außen, z.B. durch Fingermanipulation unterstützt werden kann, und ferner dadurch, daß bei der Laparoskopie das räumliche Erkennen der zu ligierenden Gefäße naturgemäß verringert ist. Beispielsweise läßt sich bei der Cholecystektomie die Arteria cystica und der Ductus cysticus insbesondere bei fettleibigen Patienten nur mit Schwierigkeit erfassen.

Zur Behebung dieser Nachteile wird ein neuartiges Klammersetzgerät der eingangs erwähnten Art vorgeschlagen, welches gemäß Kennzeichen des Hauptanspruches ausgebildet ist, wobei besondere Ausführungsformen in den Unteransprüchen aufgeführt sind.

Überraschenderweise hat es sich gezeigt, daß das betreffende Gefäß durch die Anordnung eines hin- und herbewegbaren Rückholdrahtes in den in den Klemmbacken liegenden Clip hineingezogen werden kann, wobei bei einer besonders bevorzugten Ausführungsform der Rückholdraht auch um seine Längsachse begrenzt schwenkbar ist.

Bei einer besonders bevorzugten Ausführungsform kann die Hin- und Herbewegung und/oder die Schwenkbewegung des Rückholdrahtes gegen die Kraft einer Feder ausgeführt werden, so daß der Rückholdraht unter Federwirkung in seine Ausgangslage nämlich in die direkte Nähe neben die Klemmbacken zurückgebracht werden kann.

Im folgenden soll die Erfindung anhand von Zeichnungen näher beschrieben werden; es zeigen:

Figur 1    eine Gesamtansicht des erfindungsgemäßen Klammersetzgerätes;

Figur 2    eine Einzeldarstellung der Klemmbackenausbildung des erfindungsgemäßen Klammersetzgerätes;

Figur 3    eine Darstellung des griffseitigen Endes des erfindungsgemäßen Klammersetzgerätes.

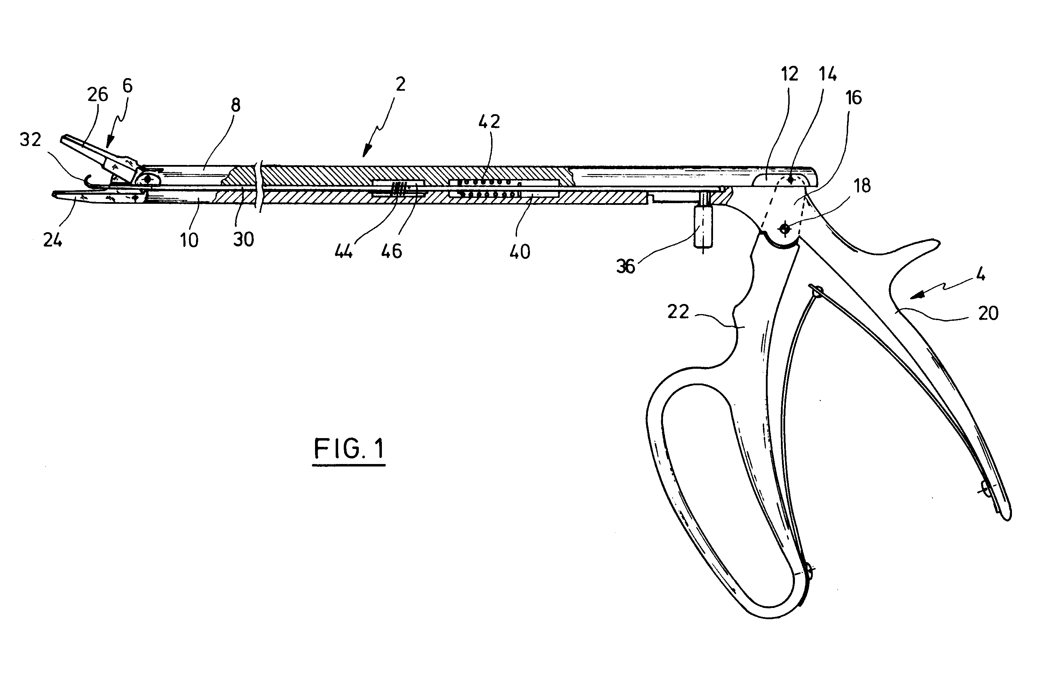

Das in Figur 1 in seiner Gesamtheit gezeigte Klammersetzgerät besteht aus einem Gestänge 2, einem distalen Griff 4 und proximalen Klemmbacken 6, wobei die Begriffe distal und proximal in bezug auf den Körper des Patienten bezogen sind.

Das Gestänge 2 besteht im einzelnen aus einer oberen Stange 8 und einer unteren Stange 10, die beide einen halbkreisförmigen Querschnitt besitzen und mit ihren Flachseiten zueinander derart angeordnet sind, daß sie ein rohrartiges Gestänge bilden. Die obere Stange 8 und die untere Stange 10 werden in ihrer begrenzten axialen Bewegung durch eine Gelenkverbindung im Bereich der Klemmbacken 6 und eine entsprechende Gelenkverbindung im Bereich des Griffes 4 geführt, wie es im folgenden im einzelnen beschrieben ist.

Es ist auch möglich, die obere Stange 8 und die untere Stange 10 durch entsprechende T-förmige oder L-förmige Vorsprünge in entsprechend geformten Nuten zu führen, wenngleich bei einer bevorzugten Ausführungsform die Führung mittels eines schwenkbaren Verbindungselementes 48 im Bereich der Klemmbacken 6 und eines entsprechenden Verbindungselementes 16 im Griffbereich völlig ausreicht.

Bei der in Figur 1 gezeigten Ausführungsform ist der erfindungswesentliche Rückholdraht 30 in einer Nut 38 in der unteren Stange 10 angeordnet und verläuft in Längsrichtung zu dieser unteren Stange 10. Der Rückholdraht 30 hat ein hakenförmiges Ende 32 mit einem nach oben offenen Haken, während das hintere Ende 34 des Rückholdrahtes im Bereich des Griffes 4 endet und in einen Betätigungshebel 36 übergeht, der aus dem Gestänge 2 herausragt. Der Betätigungshebel 36 kann je nach Wunsch stift- oder stumpfförmig oder aber auch ringförmig ausgebildet sein.

Der Rückholdraht 30 ist über den Betätigungshebel 36 in axialer Richtung verschiebbar und bei einer bevorzugten Ausführungsform durch eine Rückholfeder 42, die in einer Kammer 40 für die Rückholfeder in der unteren Stange 10 eingelassen ist, in seine Ruhestellung zurückbringbar.

Bei einer weiteren bevorzugten Ausführungsform ist der Rückholdraht 30 aus seiner Ausgangsstellung mit dem nach oben gerichteten Haken um jeweils bis zu etwa 45° beidseitig um seine Längsachse schwenkbar. Hierzu ist in einer weiteren Kammer 44 eine Rückstellfeder 46 vorgesehen, die den Haken wieder in seine senkrecht ausgerichtete Ruhestellung bringt.

Bei der in Figur 1 gezeigten Ausbildung des Griffteiles ist der äußere Griffteil 20 materialeinheitlich mit der untere Stange 10 des Griffes 4 verbunden, während der innere Griffteil 22 um eine Schwenkachse 18 des Griffteils schwenkbar mit einem Verbindungselement 16 verbunden ist, das wiederum um einen Achsstift 14 mit einer Nase 12 der oberen Stange verbunden ist. Hierdurch ergibt sich beim Anziehen des inneren Griffteils 22 eine Vorwärtsbewegung der oberen Stange 8 in axialer Richtung bei gleichzeitig sicherer Führung der betreffenden Stange.

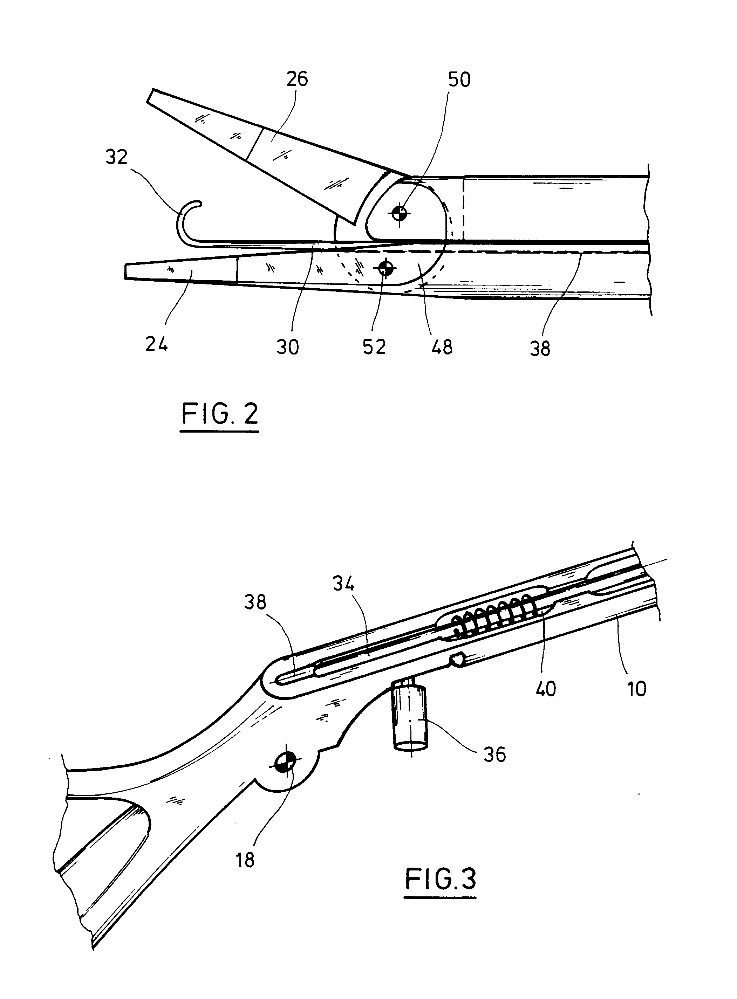

Analog ausgebildet ist die in Figur 2 in ihrer Einzelheit gezeichnete Ausführung der Klemmbakken. Bei dieser Darstellung ist die Spreizstellung der Klemmbacken der Übersichtlichkeit wegen etwas übertrieben dargestellt; für laparoskopische Eingriffe, bei denen das Gerät durch einen Trokar eingeführt wird, muß die Oberkante der oberen Klemmbacke 26 in Spreizstellung mit der Oberkante der oberen Stange 8 fluchten. Die untere Klemmbacke 24 ist materialeinheitlich mit der unteren Stange 10 ausgebildet, in welcher die Nut 38 für den Rückholdraht 30 vorgesehen ist. Die obere Klemmbacke 26 ist über ein Achsstift 50 mit dem Ende der oberen Stange 8 schwenkbar verbunden, und zwar über ein Verbindungselement 48, welches über einen weiteren Achsstift 52 in der unteren Stange 10 verankert ist.

Beim Zusammendrücken von äußerem Griffteil 20 und innerem Griffteil 22 bewegt sich die obere Stange 8 nach vorne bzw. in proximaler Richtung, wodurch die obere Klemmbacke 26 zuklappt und den vorher eingesetzten Clip zusammendrückt.

Wie in Figur 3 gezeigt, ist das hintere Ende 34 des Rückholdrahtes 30 mit einem Betätigungshebel 36 verbunden, der gegen die Kraft der Rückholfeder 40 betätigt werden kann und auch gegen die Kraft der Rückstellfeder 44, um seine Längsachse schwenkbar ist.

Bei Einsatz des Gerätes wird zuerst zwischen der unteren Klemmbacke 24 und der oberen Klemmbacke 26 ein offen ausgerichteter Clip eingesetzt und nach entsprechender Einführung des Clip-Applikators in das Operationsgebiet der Rückholdraht 30 vorgeschoben, bis er das zu ergreifende Gefäß gegebenenfalls bei unter Verschwenkung des Hakens erfaßt, worauf durch entsprechende Manipulation des Betätigungshebels 36 dieser mit dem eingefangenen Gefäß in den Maulbereich der Klemmbacken 6 gebracht und durch entsprechende Betätigung der Griffe die Klammer um das Gefäß gesetzt wird.

Die erfindungsgemäßen endoskopischen Clip-Applikatoren können nicht nur für die Cholecystektomie eingesetzt werden, sondern sind allgemein immer dann von Vorteil, wenn man Gefäße abklemmen muß, wie beispielsweise bei der selektiven proximalen Vagotomie, beim Mobilisieren von Ösophagus, bei Leberresektionen, bei der Lobektomie und Segmentresektion, bei der Lymphadenektomie und auch für Ligaturen bei der Pelviskopie.

**Bezugszeichenaufstellung**

Gestänge
2
Griff
4
Klemmbacken
6
obere Stange
8
untere Stange
10
Nase
12
Achsstift
14
Verbindungselement
16
Schwenkachse des Griffsteils
18
äußerer Griffteil
20
innerer Griffteil
22
untere Klemmbacke
24
obere Klemmbacke
26
Rückholdraht
30
vorderes Ende des R.
32
hinteres Ende des R.
34
Betätigungshebel
36
Nut für Rückholdraht
38
Kammer für Rückholfeder
40
Kammer für Rückholfeder
42
Kammer für Rückstellfeder
44
Kammer für Rückstellfeder
46
Verbindungselement für 26
48
Achsstift
50
weiterer Achsstift
52

**Patentansprüche**

1.  Klammersetzgerät für Ligatur-Clips, insbesondere für laparoskopische Operationen, bestehend aus einem rohrartigen Gestänge, an dessen einem Ende ein zweiteiliger scherenartiger Griff und an dessem entgegengesetzten Ende Klemmbacken zur Aufnahme einer Klammer vorgesehen sind, wobei der eine Griffteil des scherenartigen Griffs fest mit dem einen Teil des Gestänges verbunden ist, während der andere Griffteil ein Schließen der Klemmbacken bewirkt, wenn beide Griffteile um ihre unterhalb und senkrecht zur Längsachse des rohrartigen Gestänges liegende Schwenkachse gegen Federdruck relativ zueinander bewegt werden, **dadurch gekennzeichnet, daß** in dem rohrartigen Gestänge (2) ein Rückholdraht (30) bewegbar angeordnet ist, dessen vorderes bzw. proximales Ende (32) hakenförmig ausgebildet ist und in Ruhestellung mit nach oben offenem Haken neben den Klemmbacken (24, 26) liegt, während das hintere bzw. distale Ende (34) des Rückholdrahtes (30) im Bereich des Griffes (4) mit einem aus dem rohrartigen Gestänge (2) herausragenden Betätigungshebel (36) verbunden ist.

2. Klammersetzgerät nach Anspruch 1, **dadurch gekennzeichnet, daß** der Rückholdraht (30) unter der Wirkung einer Rückholfeder (42) in seine Ruhestellung gedrückt wird und beim Vorschieben des Betätigungshebels (36) in proximaler Richtung über die Klemmbacken (24, 26) hinausbewegbar ist.

3. Klammersetzgerät nach Anspruch 1 und 2, **dadurch gekennzeichnet, daß** der Rückholdraht (30) um seine Längsachse schwenkbar ist.

4. Klammersetzgerät nach Anspruch 1 bis 3, **dadurch gekennzeichnet, daß** der Rückholdraht (30) aus seiner Ausgangsstellung mit nach oben gerichtetem Haken um jeweils bis zu 45° beidseitig um seine Längsachse schwenkbar ist.

5. Klammersetzgerät nach Anspruch 4, **dadurch gekennzeichnet,** daß der Rückholdraht (30) aus seiner Ausgangsstellung gegen die Kraft einer Rückstellfeder (46) schwenkbar ist.

6. Klammersetzgerät nach Anspruch 1 bis 5, **dadurch gekennzeichnet**, daß

   (a) das Gestänge (2) aus einer oberen Stange (8) und einer unteren Stange (10) besteht, die beide einen halbkreisförmigen Querschnitt haben und mit ihren Flachseiten aufeinanderliegend oder zwangsgeführt in axialer Richtung begrenzt verschiebbar sind,
   (b) der äußere Griffteil (20) des scherenartigen Griffes (4) mit der unteren Stange (10) verbunden ist und im Verbindungsbereich mit dieser unterhalb der Längsachse des Gestänges (2) und quer zu dieser eine Schwenkachse (18) aufweist, um die der innere Griffteil (22) schwenkbar ist, und oberhalb der Schwenkachse mit der oberen Stange (8) verbunden ist,

   (c) die Klemmbacken (6) aus einer unteren Klemmbacke (24), die materialeinheitlich am Ende der unteren Stange (10) ausgeformt ist, und einer oberen Klemmbacke (26) bestehen, die schwenkbar an der oberen Stange (8) derart befestigt ist, daß sie bei einer Parallelverschiebung der oberen Stange (8) durch Anziehen des inneren Griffteils (22) in Richtung auf den äußeren Griffteil (20) in Schließstellung bringbar ist,

   (d) der Rückholdraht in einer axial verlaufenden Nut oder Bohrung (38) in der unteren Stange (10) geführt ist und am distalen Ende mit einem Betätigungshebel (36) versehen ist.

7. Klammersetzgerät nach Anspruch 1 bis 6, **dadurch gekennzeichnet**, daß die Rückholfeder (42) zur axialen Rückführung des Rückholdrahtes (30) in die Ruhestellung bzw. die Rückstellfeder (46) zur Schwenkung in die senkrechte Ruhestellung in Vertiefungen oder Kammern (40, 44) in der unteren Stange (10) angebracht sind.

8. Klammersetzgerät nach Anspruch 1, **dadurch gekennzeichnet, daß** der Rückholdraht (30) in seinem distalen Bereich durch eine erste axiale Nut oder Bohrung in der oberen Stange (8) und in seinem proximalen Bereich durch eine sich an die erste axiale Nut oder Bohrung anschließende zweite Nut oder Bohrung in der unteren Stange (10) geführt ist.

FIG. 1

FIG. 2

FIG.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 315 371   (CONCEPT, INC.)<br>* Anspruch 1; Figuren 2,3,5,6 *<br>– – – | 1 | A 61 B 17/12 |
| A | PATENT ABSTRACTS OF JAPAN Band 13, Nr. 115 (C-578)(3463), 20. März 1989;<br>& JP - A - 63288147 (OLYMPUS OPTICAL CO LTD) 25.11.1988<br>* Zusammenfassung *<br>– – – | 1 | |
| A | DE-U-8 808 868   (WEBA MEDIZINMECHANIK GMBH & CO.)<br>* Anspruch 1; Figuren 1-3 *<br>– – – | 1 | |
| A | US-A-4 505 272   (UTYAMYSHEV)<br>* Anspruch 1; Spalte 5, Zeilen 35-43 *<br>– – – | 1 | |
| A | WO-A-8 401 279   (UNITED SURGECIAL CORPORATION)<br>* Anspruch 1; Seite 8, Zeilen 35-38; Figuren 8,16 *<br>– – – – – | 1 | |

RECHERCHIERTE SACHGEBIETE (Int. Cl.5)

A 61 B
A 61 F

Der vorliegende Recherchenbericht wurde für alle Patentansprüche erstellt

| Recherchenort | Abschlußdatum der Recherche | Prüfer |
|---|---|---|
| Berlin | 25 Oktober 91 | PAPA E.R. |